# DEMANDE DE BREVET EUROPEEN

(11) **EP 3 252 044 A1**
(43) Date de publication de la demande: **06.12.2017**
(21) Numéro de dépôt: 17166690.2
(22) Date de dépôt: 03.03.2014
(51) Int. Cl.: C07D 307/85, A61K 31/343, A61P 29/00, A61P 35/00

(54) **PROCEDE DE PREPARATION DE SEL DE TOSYLATE DE L'ABEXINOSTAT ET FORME CRISTALLINE ASSOCIEE**

(30) Priorité: 04.03.2013 FR 1351898; 04.03.2013 US 201361772191 P
(62) Demande divisionnaire de: 14711833.5
(71) Demandeur: Pharmacyclics LLC, Sunnyvale, CA 94085 (US)
(72) Inventeur: PIMONT-GARRO, Anne, F-78000 Versailles (FR); LETELLIER, Philippe, F-45000 Orleans (FR)
(74) Mandataire: HGF Limited

(57) **Abrégé**

Procédé de préparation du Tosylate d'abexinostat de formule (II) : 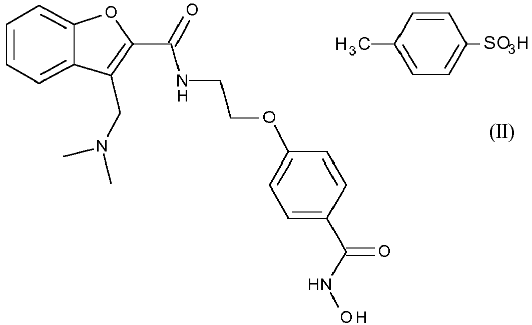 et sa forme cristalline 1 caractérisée par son diagramme de diffraction X sur poudre, son spectre Raman, et son spectre RMN à l'état solide ¹³C CP/MAS.

## Description

La présente invention concerne le tosylate de *N-*hydroxy-4-{2-[3-(*N*,*N-*diméthylaminométhyl)benzofuran-2-ylcarbonylamino]éthoxy}benzamide, ou l'un de ses solvates.

Alternativement, l'objet de l'invention concerne un sel de tosylate de l'abexinostat de formule (I) :

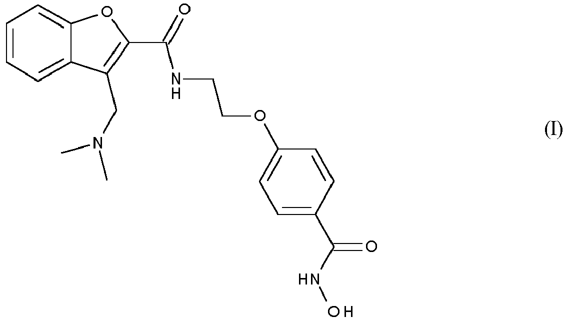

Plus particulièrement, le sel de formule (II) est visé :

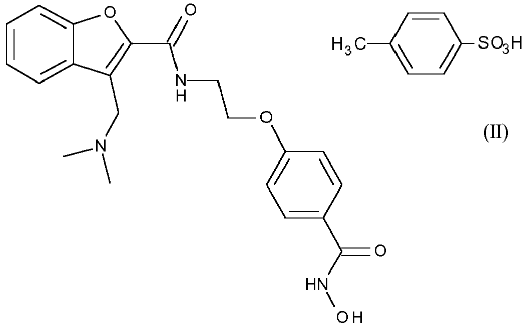

La présente invention concerne également la forme cristalline I du tosylate de *N*-hydroxy-4-{2-[3-(*N,N*-diméthylaminométhyl)benzofuran-2-ylcarbonylamino]éthoxy}benzamide, son procédé de préparation ainsi que les compositions pharmaceutiques qui la contiennent.

Le *N*-hydroxy-4-{2-[3-(*N*,*N-*diméthylaminométhyl)benzofuran-2-ylcarbonylamino] éthoxy}benzamide, aussi appelé abexinostat, est un inhibiteur des histones-déacétylases (HDAC) décrit dans la demande de brevet WO2004/092115. Il permet d'inhiber la croissance cellulaire et induit l'apoptose dans des cellules tumorales cultivées *in vitro,* et inhibe la croissance tumorale *in vivo* dans des modèles de xénogreffes (Buggy et al Mol. Cancer Ther 2006 5(5) 1309). Compte-tenu de son profil pharmacologique, l'abexinostat est destiné à être utilisé dans le traitement du cancer.

Du point de vue industriel, il est primordial de pouvoir synthétiser le composé avec une excellente pureté, et notamment sous une forme parfaitement reproductible, présentant des caractéristiques intéressantes de dissolution, de filtration, de séchage, de facilité de formulation et de stabilité permettant son stockage prolongé sans conditions particulières de température, de lumière, d'humidité ou de taux d'oxygène.

La demande de brevet WO2004/09211 décrit deux voies d'accès différentes pour obtenir l'abexinostat. Dans les deux cas, l'acide 3-méthyl-benzofuran-2-carboxylique est utilisé comme produit de départ, mais la fonctionnalisation de ce noyau central par le groupement diméthylaminométhyl en position 3 est réalisée à des étapes différentes du procédé de synthèse, en l'occurrence avant ou après le couplage du dérivé de l'acide benzofuran-2-carboxylique avec le 4-(2-aminoéthoxy)benzoate de méthyle. L'obtention du chlorhydrate d'abexinostat est spécifiquement décrite dans la demande WO2004/092115 Cependant, l'utilisation de ce sel à une échelle industrielle est délicate du fait de ses propriétés hygroscopiques.

La présente invention décrit un procédé d'obtention du tosylate d'abexinostat (4-méthylbenzènesulfonate d'abexinostat) sous une forme cristalline bien définie, parfaitement reproductible, et présentant une très bonne stabilité compatible avec les contraintes industrielles de préparation (de séchage en particulier) et de conservation des compositions pharmaceutiques.

La forme cristalline I du tosylate d'abexinostat est caractérisée par un diagramme de diffraction X sur poudre présentant les raies de diffraction suivantes (angle de Bragg 2 thêta, exprimé en degrés ±0,2°) : 6,50 ; 9,94 ; 11,35 ; 12,33 ; 14,08 ; 18,95 ; 21,08 ; 27,05. Plus particulièrement encore, la forme cristalline I du tosylate d'abexinostat est caractérisée par les raies de diffraction suivantes : 6,50 ; 9,94 ; 11,35 ; 12,33 ; 14,08 ; 18,95 ; 19,61 ; 19,96 ; 21,08 ; 22,82 ; 23,61 ; 27,05.

Plus spécifiquement, la forme cristalline I du tosylate d'abexinostat est caractérisée par le diagramme de diffraction X sur poudre ci-dessous, mesuré à l'aide d'un diffractomètre PANalytical X'Pert Pro MPD avec un détecteur X'Celerator, et exprimé en termes de position de raie (angle de Bragg 2 thêta, exprimé en degrés ±0,2°) et de distance inter-réticulaire d (exprimée en Å) :

| **Raie n°** | **Angle 2-thêta (degrés)** | **Distance inter-réticulaire (Å)** |
|---|---|---|
| 1 | 6,50 | 13,581 |
| 2 | 9,94 | 8,894 |
| 3 | 11,35 | 7,789 |
| 4 | 12,33 | 7,173 |
| 5 | 14,08 | 6,285 |
| 6 | 18,95 | 4,683 |
| 7 | 19,61 | 4,526 |
| 8 | 19,96 | 4,449 |
| 9 | 21,08 | 4,215 |
| 10 | 22,82 | 3,897 |
| 11 | 23,61 | 3,768 |
| 12 | 27,05 | 3,296 |

En outre, la forme cristalline I du tosylate d'abexinostat a été caractérisée par spectroscopie Raman. Des pics significatifs ont été observés aux positions suivantes : 940 cm⁻¹, 1088 cm⁻¹, 1132 cm⁻¹, 1242 cm⁻¹, 1360 cm⁻¹, 1608 cm⁻¹.

Alternativement, la forme cristalline I du tosylate d'abexinostat peut être caractérisée par le diagramme de diffraction X sur poudre comportant les 12 raies significatives présentées précédemment, ainsi que par un spectre Raman présentant un pic significatif à la position 1608 cm⁻¹.

Enfin, la forme cristalline I du tosylate d'abexinostat a également été caractérisée par spectroscopie RMN à l'état solide. Des pics significatifs ont été observés à 121,2 ppm, 122,1 ppm, 123,5 ppm, 126,0 ppm, 126,8 ppm, 128,2 ppm, 128,9 ppm, 143,4 ppm, 144,6 ppm, 153,8 ppm, 159 ppm, 161,2 ppm et 162,1 ppm.

Plus précisément, les spectres ¹³C CP/MAS (Cross Polarization Magic Angle Spinning) présentent les pics suivants (exprimés en ppm ± 0,2 ppm) :

| **Pic n°** | **Déplacement chimique (ppm)** | **Pic n°** | **Déplacement chimique (ppm)** |
|---|---|---|---|
| 1 | 162,1 | 10 | 126,0 |
| 2 | 161,2 | 11 | 123,5 |
| 3 | 159,0 | 12 | 122,1 |
| 4 | 153,8 | 13 | 121,3 |
| 5 | 144,6 | 14 | 65,9 |
| 6 | 143,4 | 15 | 50,6 |
| 7 | 128,9 | 16 | 46,9 |
| 8 | 128,2 | 17 | 45,0 |
| 9 | 126,8 | 18 | 21,9 |

L'invention s'étend également au procédé de préparation de la forme cristalline I du tosylate d'abexinostat, caractérisé en ce que l'abexinostat est cristallisé dans un milieu polaire en présence d'acide *para*-toluènesulfonique. De manière préférentielle, le milieu polaire est constitué d'un ou plusieurs solvants choisis parmi l'eau, les alcools, les cétones et les esters, étant entendu que :
- par "alcools", on entend les alcools C₁-C₆ tels que le méthanol, l'éthanol, le propanol, l'isopropanol, le butanol, l'isobutanol, le pentanol, le 2-pentanol, le 3-pentanol, l'isopentanol, l'hexanol,
- par "cétones", on entend les cétones C₃-C₆ telles que l'acétone, la méthyléthyl cétone, la 2-pentanone, la 3-pentanone, la 3-méthyl-2-butanone, la 2-hexanone, la 3-hexanone, l'éthylisopropylcétone, la méthylisopropylcétone, la 2,2-diméthyl-3-butanone,
- par "esters", on entend les esters C₃-C₈ tels que le formate d'éthyle, le formate d'isopropyle, l'acétate d'éthyle, l'acétate de propyle, l'acétate d'isopropyle, l'acétate de butyle, l'acétate d'isobutyle, l'acétate de *tert*-butyle, l'acétate de pentyle, l'acétate d'isopentyle, l'acétate d'hexyle.

Les alcools préférés sont l'éthanol et l'isopropanol. Parmi les solvants préférés, on retiendra également l'acétone et la méthyléthylcétone pour les cétones, et l'acétate d'éthyle pour les esters.

Alternativement, le milieu polaire est un mélange binaire dont l'un des constituants est l'eau. Plus préférentiellement encore, le milieu polaire est un mélange binaire choisi parmi : acétone/eau, éthanol/eau, isopropanol/eau et méthyléthylcétone/eau.

Dans le procédé de cristallisation selon l'invention, on peut utiliser l'abexinostat (base libre) obtenu par n'importe quel procédé.

L'invention s'étend également à un autre procédé de préparation de la forme cristalline I du tosylate d'abexinostat, dans lequel la cristallisation est amorcée par une très petite quantité de la forme cristalline I du tosylate d'abexinostat.
Dans ce second procédé de cristallisation selon invention, on peut aussi utiliser l'abexinostat (base libre) obtenu par n'importe quel procédé.

L'obtention de la forme cristalline I du tosylate d'abexinostat a pour avantage de permettre la préparation de formulations pharmaceutiques ayant une composition constante et reproductible, présentant de bonnes caractéristiques de dissolution et de stabilité, ce qui est particulièrement avantageux lorsque les formulations sont destinées à l'administration orale. Plus précisément, l'utilisation de la forme cristalline I du tosylate d'abexinostat est particulièrement intéressante sur le plan industriel compte-tenu de sa faible hygroscopicité.

La forme cristalline I du tosylate d'abexinostat est destinée au traitement du cancer, et plus particulièrement au traitement d'un carcinome, d'une tumeur, d'un néoplasme, d'un lymphome, d'un mélanome, d'un gliome, d'un sarcome, ou d'un blastome.

L'invention s'étend aussi aux compositions pharmaceutiques renfermant comme principe actif un sel de tosylate de l'abexinostat, et plus particulièrement encore la forme cristalline I du tosylate d'abexinostat, avec un ou plusieurs excipients inertes, non toxiques et appropriés. Parmi les compositions pharmaceutiques selon l'invention, on pourra citer plus particulièrement celles qui conviennent pour l'administration orale, parentérale (intraveineuse ou sous-cutanée), nasale, les comprimés simples ou dragéifiés, les granulés, les comprimés sublinguaux, les gélules, les tablettes, les suppositoires, les crèmes, les pommades, les gels dermiques, les préparations injectables, les suspensions buvables et les pâtes à mâcher.

Les compositions pharmaceutiques administrées par voie orale sont préférées.

La posologie utile varie selon le sexe, l'âge et le poids du patient, la voie d'administration, la nature du cancer et des traitements éventuellement associés et la posologie utile s'échelonne entre 20 mg et 480 mg de *N*-hydroxy-4-{2-[3-(*N*,*N-*diméthylaminométhyl)benzo-furan-2-ylcarbonylamino]éthoxy}benzamide exprimé en base libre par jour.

Les exemples ci-dessous illustrent l'invention, mais ne la limitent en aucune façon.

### Exemple 1: Procédé d'obtention de la forme cristalline I du tosylate d'abexinostat

1,66 kg d'abexinostat (base libre) sont placés dans 9,48 kg d'un mélange d'isopropanol/eau (50/50 masse/masse) à température ambiante. L'acide *para*-toluènesulfonique monohydraté (0,83 kg) est ajouté dans 2,36 kg d'eau à température ambiante. Le milieu est ensuite chauffé à 75°C pendant 30 minutes avant d'être refroidi à 0°C. Lorsque la cristallisation est totale, la suspension est filtrée à 20°C. Après séchage, la forme cristalline I du tosylate d'abexinostat est obtenue avec un rendement d'environ 85% et une pureté supérieure à 99%. Le solide a été caractérisé par le diagramme de diffraction X sur poudre, le spectre Raman et le spectre RMN détaillés aux Exemples 3-6 suivants.

### Exemple 2: Procédé d'obtention de la forme cristalline I du tosylate d'abexinostat (ensemencement)

33,9 kg d'abexinostat (base libre) sont placés dans 170 kg d'un mélange d'isopropanol/eau (45,6/54,4 masse/masse) à température ambiante. Une solution constituée d'acide *para-*toluènesulfonique monohydraté (17,06 kg) dans l'eau (24,1 kg) est ajoutée. Le milieu est ensuite chauffé à 70-75°C, refroidi et amorcé avec 1,935 kg de forme cristalline I de tosylate d'abexinostat. La suspension est ensuite filtrée à 20°C. Après séchage, la forme cristalline I du tosylate d'abexinostat est obtenue avec un rendement d'environ 86% et une pureté supérieure à 99%. Le solide a été caractérisé par le diagramme de diffraction X sur poudre, le spectre Raman et le spectre RMN détaillés aux Exemples 3-6 suivants.

### Exemple 3: Forme cristalline I du tosylate d'abexinostat (diagramme de diffraction X sur poudre)

L'enregistrement des données a été effectué sur un diffractomètre PANalytical X'Pert Pro MPD avec un détecteur X'Celerator dans les conditions suivantes :
- Tension 45 kV, intensité 40 mA,
- Montage thêta/thêta,
- Anode : cuivre,
- Longueur d'onde K alpha-1 : 1,54060 Å,
- Longueur d'onde K alpha-2 : 1,54443 Å,
- Rapport K alpha-2/ K alpha-1 : 0,5
- Mode de mesure : continu de 3° à 55° (angle de Bragg 2 thêta) avec une incrémentation de 0,017°,
- Temps de mesure par pas : 35,53 s.

Le diagramme de diffraction X sur poudre de la forme I du tosylate d'abexinostat obtenu selon le procédé de l'Exemple 1 ou 2 est exprimé en terme de position de raie (angle de Bragg 2 thêta, exprimé en degrés ±0,2°), de distance inter-réticulaire d (exprimée en Å) et d'intensité relative (exprimée en pourcentage par rapport à la raie la plus intense). Les raies significatives ont été rassemblées dans le tableau suivant :

| **Raie n°** | **Angle 2-thêta (degrés)** | **Distance inter-réticulaire (Å)** | **Intensité relative (%)** |
|---|---|---|---|
| 1 | 6,50 | 13,581 | 75,6 |
| 2 | 9,94 | 8,894 | 58,4 |
| 3 | 11,35 | 7,789 | 19,1 |
| 4 | 12,33 | 7,173 | 23,7 |
| 5 | 14,08 | 6,285 | 33,1 |
| 6 | 18,95 | 4,683 | 100 |
| 7 | 19,61 | 4,526 | 53,9 |
| 8 | 19,96 | 4,449 | 50,9 |
| 9 | 21,08 | 4,215 | 93,5 |
| 10 | 22,82 | 3,897 | 28,5 |
| 11 | 23,61 | 3,768 | 32,6 |
| 12 | 27,05 | 3,296 | 16,0 |

### Exemple 4: Forme cristalline I du tosylate d'abexinostat (maille cristalline)

Une solution saturée de tosylate d'abexinostat dans le 2,2,2-trifluoroéthanol est préparée par agitation d'une suspension pendant 24 heures à température ambiante, suivie d'une filtration. On verse ensuite 1 mL de cette solution saturée dans un vial HPLC de 1,8 mL auquel on ajoute 0,25 mL d'eau. La solution est maintenue à température ambiante pendant 75 minutes. Après centrifugation puis séchage, le solide est isolé pour analyse. Parmi les cristaux obtenus, un cristal de qualité suffisante est prélevé pour analyse par diffraction X sur monocristal.

La structure cristalline a été déterminée sur le monocristal précédent à l'aide d'un diffractomètre Bruker Kappa CCD équipé d'un générateur FR590 à anticathode molybdène ((λ-MoKα1 = 0,7093 Å) avec un domaine angulaire compris entre 2° et 27,5° en θ. Les paramètres suivants ont été établis :
- maille cristalline triclinique,
- paramètres de maille : a = 10,467 Å, b = 14,631 Å, c = 20,159 Å, α = 73,971°, β = 79,040°, γ = 72,683°
- groupe d'espace : P -1
- nombre de molécules dans la maille : 4
- volume de la maille : Vₘₐᵢₗₗₑ = 2813,0 Å³
- densité : d = 1,345 g/cm³.

### Exemple 5: Forme cristalline I du tosylate d'abexinostat (spectre Raman)

La forme I du tosylate d'abexinostat a été caractérisée par spectroscopie Raman. Les spectres ont été enregistrés en mode réflexion diffuse (Raman Station 400, PerkinElmer) avec un laser à 785 nm. Le signal a été enregistré par un détecteur CCD. Le décalage en longueur d'onde dépend de la matière et lui est caractéristique, ce qui permet une analyse de la composition chimique et de l'agencement moléculaire de l'échantillon étudié. Les spectres ont été acquis avec une puissance maximale (100% de la capacité du laser), une taille de spot de 100 µm, vingt expositions de 2 secondes et une résolution spectrale de 2 cm⁻¹. La gamme spectrale explorée s'échelonne entre 0 et 3278 cm⁻¹.

Des pics significatifs ont été observés aux positions suivantes : 940 cm⁻¹, 1088 cm⁻¹, 1132 cm⁻¹, 1242 cm⁻¹, 1360 cm⁻¹, 1608 cm⁻¹.

### Exemple 6: Forme cristalline I du tosylate d'abexinostat (spectre RMN Solide)

La forme I du tosylate d'abexinostat a également été caractérisée par spectroscopie RMN à l'état solide. Les spectres ¹³C RMN ont été enregistrés à température ambiante à l'aide d'un spectromètre Bruker SB Avance avec une sonde de type 4 mm CP/MAS SB VTN dans les conditions suivantes :
- Fréquence : 125,76 MHz,
- Largeur spectrale : 40 kHz,
- Vitesse de rotation de l'échantillon à l'angle magique : 10 kHz,
- Séquence d'impulsion : CP (Cross Polarization) avec découplage SPINAL64 (puissance de découplage de 80 kHz),
- Délai de répétitions : 10 s,
- Temps d'acquisition : 35 ms,
- Temps de contact : 4 ms
- Nombre de scans : 4096.

Une fonction d'apodisation (« 5 Hz line broadening ») est appliquée au signal recueilli avant la transformée de Fourier. Les spectres ainsi obtenus ont été référencés par rapport à un échantillon d'adamantane (le pic de plus haute fréquence de l'adamantane a pour déplacement chimique 38,48 ppm).

Les pics observés ont été rassemblés dans le tableau suivant (exprimés en ppm ± 0,2 ppm) :

| **Pic n°** | **Déplacement chimique (ppm)** | **Pic n°** | **Déplacement chimique (ppm)** |
|---|---|---|---|
| 1 | 162,1 | 10 | 126,0 |
| 2 | 161,2 | 11 | 123,5 |
| 3 | 159,0 | 12 | 122,1 |
| 4 | 153,8 | 13 | 121,3 |
| 5 | 144,6 | 14 | 65,9 |
| 6 | 143,4 | 15 | 50,6 |
| 7 | 128,9 | 16 | 46,9 |
| 8 | 128,2 | 17 | 45,0 |
| 9 | 126,8 | 18 | 21,9 |

### Exemple 7: Composition pharmaceutique

Formule de préparation pour 1000 comprimés dosés à 100 mg d'abexinostat (exprimé en équivalent base) :

| | |
|---|---|
| Tosylate d'abexinostat | 143,4 g |
| Lactose monohydrate | 213,1 g |
| Stéarate de Magnésium | 2,5 g |
| Amidon de maïs | 75 g |
| Maltodextrine | 50 g |
| Silice colloïdale anhydre | 1 g |
| Carboxymethyl cellulose sodique | 15 g |

### Exemple 8: Hygroscopie

L'hygroscopicité de la forme I du tosylate d'abexinostat a été évaluée par gravimétrie d'adsorption de vapeur d'eau (DVS - *Dynamic Vapor Sorption*). Un échantillon de 5 à 10 mg de la substance médicamenteuse, pesé avec précision, a été disposé dans une cuve d'échantillon DVS fonctionnant à 25°C sous humidité contrôlée. La variation de masse a été enregistrée au cours d'un séchage sous 0 pourcent HR (humidité relative) et durant deux cycles subséquents d'augmentation et de diminution linéaires de l'humidité relative dans la plage de 0-90 pourcent HR à une vitesse de 10 pourcent par heure. L'humidité relative a été maintenue constante lorsqu'elle a atteint soit 0 soit 90 pourcent HR, jusqu'à ce que la variation de masse soit inférieure à 0,002 pourcent par minute, avec une limite en temps de 15h.
Une augmentation en poids inférieure à 0,5% a été détectée par analyse DVS après exposition d'un échantillon à des humidités relatives comprises entre 0% et 90% à 25°C.

## Revendications

1. Procédé de préparation de tosylate d'abexinostat, comprenant l'étape consistant à cristalliser de l'abexinostat en présence d'acide para-toluènesulphonique dans un milieu polaire, le milieu polaire étant un mélange binaire d'isopropanol et d'eau.

2. Procédé selon la revendication 1, le milieu polaire étant un mélange 50/50 masse/masse d'isopropanol et d'eau.

3. Procédé selon la revendication 1, le milieu polaire étant un mélange 45,6/54,4 masse/masse d'isopropanol et d'eau.

4. Procédé selon l'une quelconque des revendications précédentes, comprenant en outre l'étape consistant à ensemencer la cristallisation avec la forme cristalline I de tosylate d'abexinostat.

5. Procédé selon l'une quelconque des revendications 1 à 4, la cristallisation résultant en la formation de la forme cristalline I de tosylate d'abexinostat, **caractérisé en ce qu'**elle a un diagramme de diffraction des rayons X sur poudre ayant les lignes de diffraction suivantes (angle de Bragg 2 thêta, exprimé en degrés ±0,2°) : 6,50, 9,94, 11,35, 14,08, 18, 95, 21, 08, 27,05.

6. Procédé selon la revendication 5, la cristallisation résultant en la formation de la forme cristalline I de tosylate d'abexinostat, **caractérisé en ce qu'**elle a un diagramme de diffraction des rayons X sur poudre ayant les lignes de diffraction suivantes (angle de Bragg 2 thêta, exprimé en degrés ±0,2°) : 6,50, 9,94, 11,35, 12,33, 14,08, 18,95, 19,96, 21,08, 22,82, 23,61, 27,05.

7. Procédé selon l'une quelconque des revendications 1 à 4, la cristallisation résultant en la formation de la forme cristalline I de tosylate d'abexinostat, **caractérisé en ce qu'**elle a le diagramme de diffraction des rayons X sur poudre suivant exprimé en fonction de la position de ligne (angle de Bragg 2, exprimé en degrés ±0,2°) et de la distance interréticulaire d (exprimée en A) :
| **Ligne n°** | **Angle 2 thêta (degrés)** | **Distance interréticulaire (Å)** |
|---|---|---|
| 1 | 6,50 | 13,581 |
| 2 | 9,94 | 8,894 |
| 3 | 11,35 | 7,789 |
| 4 | 12,33 | 7,173 |
| 5 | 14,08 | 6,285 |
| 6 | 18,95 | 4,683 |
| 7 | 19,61 | 4,526 |
| 8 | 19,96 | 4,449 |
| 9 | 21,08 | 4,215 |
| 10 | 22,82 | 3,897 |
| 11 | 23,61 | 3,768 |
| 12 | 27,05 | 3,296 |

8. Procédé selon l'une quelconque des revendications 1 à 4, la cristallisation résultant en la formation de la forme cristalline I de tosylate d'abexinostat, **caractérisé en ce qu'**elle a un spectre de Raman ayant un pic significatif à la position 1 608 cm⁻¹.

9. Procédé selon la revendication 8, la cristallisation résultant en la formation de la forme cristalline I de tosylate d'abexinostat, **caractérisé en ce qu'**elle a un spectre de Raman ayant des pics significatifs aux positions 940 cm⁻¹, 1 088 cm⁻¹, 1 132 cm⁻¹, 1 242 cm⁻¹, 1 360 cm⁻¹, 1 608 cm⁻¹.

10. Procédé selon l'une quelconque des revendications 1 à 4, la cristallisation résultant en la formation de la forme cristalline I de tosylate d'abexinostat, **caractérisé en ce qu'**elle a un spectre ¹³C CP/MAS RMN à l'état solide ayant les pics suivants (exprimés en ppm ± 0,2 ppm) : 121,2 ppm, 122,1 ppm, 123,5 ppm 126,0 ppm, 126,8 ppm, 128,2 ppm, 128,9 ppm, 143,4 ppm, 144,6 ppm, 153,8 ppm, 159 ppm, 161,2 ppm et 162,1 ppm.

11. Procédé selon la revendication 10, la cristallisation résultant en la formation de la forme cristalline I de tosylate d'abexinostat, **caractérisé en ce qu'**elle a un spectre ¹³C CP/MAS RMN à l'état solide ayant les pics suivants (exprimés en ppm ± 0,2 ppm) :
| **Pic n°** | **Déplacement chimique (ppm)** | **Pic n°** | **Déplacement chimique (ppm)** |
|---|---|---|---|
| 1 | 162,1 | 10 | 126,0 |
| 2 | 161,2 | 11 | 123,5 |
| 3 | 159,0 | 12 | 122,1 |
| 4 | 153,8 | 13 | 121,3 |
| 5 | 144,6 | 14 | 65,9 |
| 6 | 143,4 | 15 | 50,6 |
| 7 | 128,9 | 16 | 46,9 |
| 8 | 128,2 | 17 | 45,0 |
| 9 | 126,8 | 18 | 21,9 |
